# EUROPÄISCHE PATENTANMELDUNG

(11) **EP 1 561 750 A1**
(43) Veröffentlichungstag der Anmeldung: **10.08.2005**
(21) Anmeldenummer: 05002337.3
(22) Anmeldetag: 04.02.2005
(51) Int. Cl.: C07D 303/06, C07F 15/00

(54) **Verfahren zur asymmetrischen Epoxidierung von Olefinen**

(30) Priorität: 05.02.2004 DE 102004005725; 19.05.2004 DE 102004024709
(71) Anmelder: LANXESS Deutschland GmbH, 51368 Leverkusen (DE)
(72) Erfinder: Mägerlein, Wolfgang, Dr., 50733 Köln (DE); Beller, Matthias, Prof. Dr., 18211 Ostseebad Nienhagen (DE); Döbler, Christian, Dr., 18107 Lichtenhagen-Dorf (DE); Tse, Man-Kin, Dr., 18055 Rostock (DE); Bhor, Santosh, 18059 Rostock (DE); Klawonn, Markus, 18106 Rostock (DE)

(57) **Zusammenfassung**

Die vorliegende Erfindung betrifft ein Verfahren zur asymmetrischen Epoxidierung von Olefinen mit Katalysatoren auf Basis von Rutheniumkomplexen, sowie die dafür geeigneten Rutheniumkomplexe selbst.

## Beschreibung

Die vorliegende Erfindung betrifft ein Verfahren zur asymmetrischen Epoxidierung von Olefinen mit Katalysatoren auf Basis von Rutheniumkomplexen, sowie die dafür geeigneten Rutheniumkomplexe selbst.

Olefine sind gut verfügbare und preisgünstige Rohstoffe für industrielle Anwendungen. Durch Oxidation lassen sie sich in Epoxide überführen, die ihrerseits als vielseitige Zwischenstufen bei der Synthese von Wirkstoffen sowie Feinchemikalien (Kosmetik-, Polymerindustrie etc.) von großer Bedeutung sind. Besonders wertvoll hierbei sind chirale Epoxide, da sich aus Ihnen zahlreiche Strukturelemente, wie z.B. chirale Diole oder chirale Aminoalkohole, erschließen lassen, welche besonders in Naturstoffen und biologisch aktiven Wirkstoffen häufig vorkommen. Die effizienteste und ökonomischste Methode zur Synthese chiraler Epoxide ist die katalytische, asymmetrische Epoxidierung von Olefinen.

Durch Mezetti et al. (Green Chemistry **1999**, *1*, 39-41; *Organometallics* **2000**, *19*, 4117-4126) ist bekannt, dass Olefine in Gegenwart von Ruthenium-Katalysatoren und mit Wasserstoffperoxid als Oxidationsmittel epoxydiert werden können, allerdings sind die Enantioselektivitäten mit maximal 42 % unbefriedigend und die Ausbeuten in den meisten Fällen für technische Applikationen zu niedrig.

Andere Ruthenium-basierte Systeme, wie z.B. chirale Schiff-Base-Komplexe (Kureshi et al., *J. Mol. Catal. A: Chemical* **1997**, *124*, 91-97) sind bezüglich Ausbeute und Stereoselektivität ebenfalls recht ineffizient.

Ein weiteres Verfahren wurde von Nishiyama et al. beschrieben (*Chem. Commun.* **1997,** 1863-1864). Durch Verwendung von Ruthenium-pyridin-2,6-dicarboxylat-Komplexen mit chiralen Bis(oxazolinyl)pyridin-Liganden wurden bei der Epoxidierung von (*E*)-Stilben Enantioselektivitäten von 74 % bei Ausbeuten bis zu 63 % erzielt. Nachteilig bei diesem Verfahren ist jedoch, dass als Reoxidationsmittel Bis(acetoxy)iodbenzol eingesetzt werden muss, das aufgrund seines hohen Preises für technische Prozesse unattraktiv ist.

Es besteht daher weiterhin das Bedürfnis, ein allgemeines und effizientes Verfahren zur asymmetrischen Epoxidierung von Olefinen zu entwickeln, das sich sowohl durch hohe Chemo- und Enantioselektivitäten auszeichnet als auch gute Produktausbeuten liefert. Gleichzeitig ist die Verwendung eines sowohl preisgünstigen als auch umweltfreundlichen Oxidationsmittels ein aus technischer Sicht anzustrebendes Ziel.

Es wurde nun ein Verfahren zur Herstellung von stereoisomerenangereicherten Verbindungen der Formel (I) gefunden, 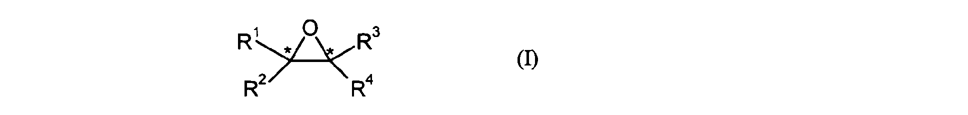 in der
- "*": für ein (R)- oder (S)- konfiguriertes Kohlenstoffatom steht und R¹, R², R³ und R⁴ jeweils unabhängig voneinander für Wasserstoff, Alkyl, Aryl, Arylalkyl, Halogenalkyl oder Reste der Formel (IIa) bis (IIf) stehen

A-B-D-E (IIa) A-E (IIb)

A-SO₂-E (IIc) A-B-SO₂R⁶ (IId)

A-SO₃W (IIe) A-COW (IIf)
wobei in den Formeln (IIa) bis (IIf)
- A: fehlt oder für einen Alkylen- oder Halogenalkylenrest steht und
- B: fehlt oder für Sauerstoff oder NR⁵ steht, wobei
- R⁵: für Wasserstoff, Arylalkyl oder Aryl steht und
- D: für eine Carbonyl-Gruppe steht und
- E: für R⁶, OR⁶, NHR⁷ oder N(R⁷)₂ steht,
wobei
- R⁶: für Alkyl, Arylalkyl oder Aryl und
- R⁷: jeweils unabhängig für Alkyl, Arylalkyl oder Aryl steht oder N(R⁷)₂ zusammen für einen cyclischen Aminorest mit 4 bis 12 Kohlenstoffatomen steht und
- W: für OH, NH₂, oder OM steht, wobei M ein Alkalimetallion, ein halbes Äquivalent eines Erdalkalimetallions, ein Ammoniumion oder ein organisches Ammoniumion steht

Oder jeweils zwei der Reste R¹, R², R³ und R⁴ zusammen Teil eines 3 bis 7-gliedrigen Cyclus sind, der insgesamt 3 bis 16 Kohlenstoffatome umfasst das dadurch gekennzeichnet ist, dass Verbindungen der Formel (III), 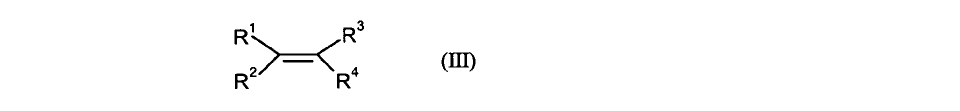 in der R¹, R², R³ und R⁴ jeweils unabhängig voneinander die vorstehend genannte Bedeutung besitzen,
mit Verbindungen der Formel (IV) umgesetzt werden,

R⁸-OOH (IV)

in der R⁸ für Wasserstoff, Alkyl oder Arylalkyl steht
wobei die Umsetzung in Gegenwart eines Rutheniumkomplexes erfolgt, der als Liganden sowohl Verbindungen der Formel (V) trägt 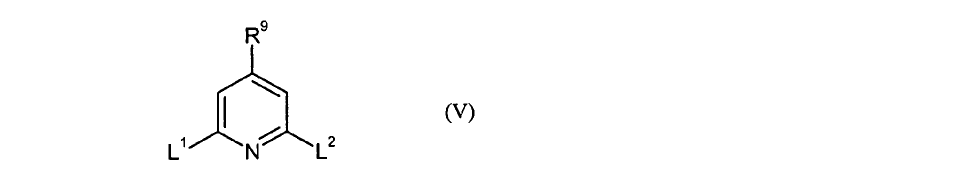 in der R⁹ für Wasserstoff, Halogen, Hydroxy, Hydroxycarbonyl, Alkoxycarbonyl, Alkoxy, Alkyl, Arylalkyl oder Aryl steht und
L¹ und L² jeweils unabhängig für Reste der Formel (VI-a) und/oder für Reste der Formel (VI-b), bevorzugt jedoch jeweils identisch für Reste der Formel (VI-a) oder für Reste der Formel (VI-b) stehen 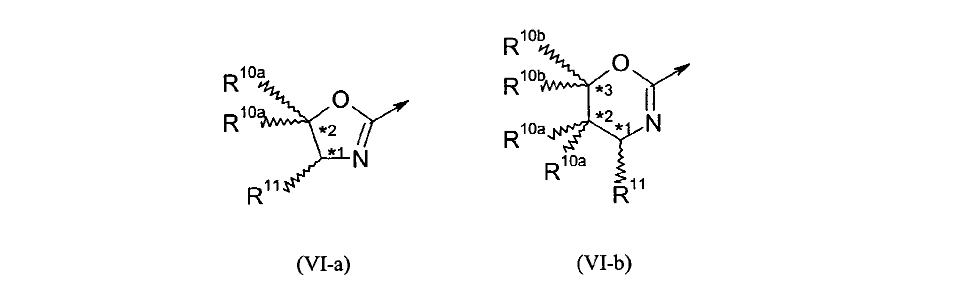 in der "*1" und/oder "*2" und/oder "*3" ein asymmetrisches Kohlenstoffatom in (R) oder (S)-Konfiguration ist, bevorzugt in der "*1" ein asymmetrisches Kohlenstoffatom in (R) oder (S)-Konfiguration ist und "*2" und/oder "*3" ebenfalls ein solches Kohlenstoffatom sein kann, der Pfeil die Bindungsstelle zum zentralen Pyridincyclus zeigt und R^{10a}, R^{10b} und R¹¹ unabhängig voneinander für Alkyl, Alkoxyalkyl, Trialkylsiloxyalkyl, Alkoxycarbonyl, Arylalkyl oder Aryl steht oder R^{10a} und R¹¹ oder R^{10a} und R^{10b} Teil eines cyclischen Restes mit insgesamt 5 bis 16 Kohlenstoffatomen sind und R¹¹ und/oder jeweils ein oder beide Reste R^{10a} und/oder R^{10b}, bevorzugt jeweils ein oder beide Reste R^{10a} und/oder R^{10b} darüber hinaus auch für Wasserstoff stehen kann
**als auch** Verbindungen der Formel (VII) trägt 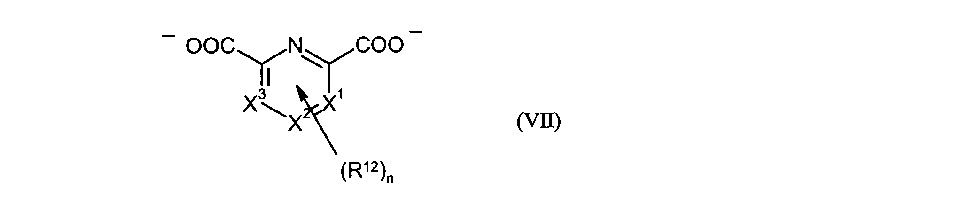 in der
- X¹, X² und X³: jeweils unabhängig voneinander für N, CH Bzw. CR¹² stehen und
- R¹²: für Wasserstoff, Halogen, Hydroxy, Hydroxycarbonyl, Alkoxycarbonyl, Alkoxy, Alkoxyalkyl, Arylalkyl oder Aryl steht und
- n: für = 1, 2 oder 3, bevorzugt für 0, oder 1 und besonders bevorzugt für 0 steht.

Stereoisomerenangereichert bedeutet im Rahmen der Erfindung dass ein Diastereomer oder Enantiomer in einem höheren relativen Anteil vorliegt als die anderen Diastereomere bzw. das andere Enantiomer.

Der Rahmen der Erfindung umfasst alle oben stehenden und im Folgenden aufgeführten, allgemeinen oder in Vorzugsbereichen genannten Restedefinitionen, Parameter und Erläuterungen in beliebiger Kombination untereinander, also auch zwischen den jeweiligen Bereichen und Vorzugsbereichen.

Aryl steht im Rahmen der Erfindung sofern nicht gesondert vermerkt bevorzugt für carbocyclische aromatische Reste mit 6 bis 24 Gerüstkohlenstoffatomen oder heteroaromatische Reste mit 5 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können. Weiterhin können die carbocyclischen aromatischen Reste oder heteroaromatische Reste mit bis zu fünf gleichen oder verschiedenen Substituenten pro Cyclus substituiert sein, ausgewählt aus der Gruppe Hydroxy, Fluor, Nitro, Cyano, freies oder geschütztes Formyl, C₁-C₁₂-Alkyl, C₅-C₁₄-Aryl, C₆-C₁₅-Arylalkyl, -PO-[(C₁-C₈)-Alkyl]₂, -PO-[(C₅-C₁₄)-Aryl]₂, -PO-[(C₁-C₈)-Alkyl)(C₅-C₁₄)-Aryl)], Tri(C₁-C₈-alkyl)siloxyl oder Resten der Formeln (IIa) bis (IIf). Gleiches gilt für den Arylteil eines Arylalkyl-Restes.

Beispielsweise steht Aryl besonders bevorzugt für Phenyl, Naphthyl oder Anthracenyl, das gegebenenfalls einfach, zweifach oder dreifach durch Reste substituiert ist, die jeweils unabhängig voneinander ausgewählt sind aus der Gruppe C₁-C₆-Alkyl, C₅-C₁₄-Aryl, C₁-C₆-Alkoxy, C₁-C₆-Alkoxycarbonyl, Halogen, Hydroxy, Nitro oder Cyano.

**Alkyl** bzw. **Alkylen** bzw. **Alkoxy** steht im Rahmen der Erfindung sofern nicht gesondert vermerkt bevorzugt jeweils unabhängig für einen geradkettigen, cyclischen, verzweigten oder unverzweigten Alkyl- bzw. Alkylen- bzw. Alkoxy-Rest, der gegebenenfalls durch C₁-C₄-Alkoxy-Reste weiter substituiert sein kann. Gleiches gilt für den Alkylenteil eines Arylalkyl-Restes.

Beispielsweise steht Alkyl besonders bevorzugt für Methyl, Ethyl, n-Propyl, Isopropyl, n-Butyl, tert.-Butyl, n-Pentyl, Cyclohexyl und n-Hexyl, n-Heptyl, n-Octyl, iso-Octyl, n-Decyl und n-Dodecyl.

Beispielsweise steht Alkylen bevorzugt für Methylen, 1,1-Ethylen, 1,2-Ethylen, 1,1-Propylen, 1,2-Propylen, 1,3-Propylen, 1,1-Butylen, 1,2-Butylen, 2,3-Butylen und 1,4-Butylen, 1,5-Pentylen, 1,6-Hexylen, 1,1-Cyclohexylen, 1,4-Cyclohexylen, 1,2-Cyclohexylen und 1,8-Octylen.

Beispielsweise steht Alkoxy bevorzugt für Methoxy, Ethoxy, Isopropoxy, n-Propoxy, n-Butoxy, tert.-Butoxy und Cyclohexyloxy.

Arylalkyl steht im Rahmen der Erfindung sofern nicht gesondert vermerkt bevorzugt jeweils unabhängig für einen geradkettigen, cyclischen, verzweigten oder unverzweigten Alkylrest, der einfach oder mehrfach, besonders bevorzugt einfach durch Arylreste gemäß vorstehender Definition substituiert ist.

**Halogenalkyl** bzw. **Halogenalkylen** steht im Rahmen der Erfindung sofern nicht gesondert vermerkt bevorzugt jeweils unabhängig für einen geradkettigen, cyclischen, verzweigten oder unverzweigten Alkylrest, der mit einem, mehreren oder vollständig mit Halogenatomen unabhängig voneinander ausgewählt aus der Gruppe Fluor , Chlor, Brom und Iod substituiert sein kann.

Beispielsweise steht C₁-C₈-Halogenalkyl besonders bevorzugt für Trifluormethyl, Trichlormethyl, 2,2,2-Trifluorethyl, Pentafluorethyl und Nonafluorbutyl, C₁-C₈-Fluoralkyl für Trifluormethyl, 2,2,2-Trifluorethyl, Pentafluorethyl und Nonafluorbutyl.

Geschütztes Formyl bedeutet einen Formyl-Rest, der durch Überführung in ein Aminal, Acetal oder ein gemischtes Aminalacetal geschützt ist, wobei die Aminale, Acetale und gemischten Aminalacetale acyclisch oder cyclisch sein können.

Im Folgenden werden bevorzugte Verbindungen der Formeln (I), (IV), (V) und (VII) definiert.

In Formel (I) stehen bevorzugt R¹, R², R³ und R⁴ jeweils unabhängig voneinander für Wasserstoff, C₁-C₈-Alkyl, C₅-C₁₄-Aryl, C₆-C₁₅-Arylalkyl, C₁-C₈-Halogenalkyl oder es sind jeweils zwei der Reste R¹, R², R³ und R⁴ zusammen Teil eines 3 bis 7-gliedrigen Cyclus, der insgesamt 3 bis 16 Kohlenstoffatome umfasst.

Weiter bevorzugt sind dabei Verbindungen, in denen mindestens ein, noch weiter bevorzugt mindestens zwei Reste aus R¹, R², R³ und R⁴ für Wasserstoff stehen.

In Formel (IV) steht R⁸ bevorzugt für Wasserstoff oder C₃-C₆-Alkyl, besonders bevorzugt für Wasserstoff oder tert.-Butyl und ganz besonders bevorzugt für Wasserstoff.

In Formel (V) steht R⁹ für bevorzugt Wasserstoff, Halogen, C₁-C₄-Alkoxy, C₁-C₄-Alkyl, Phenyl, besonders bevorzugt für Wasserstoff oder Phenyl.

Weiterhin steht in Formel (V) L¹ und L² jeweils identisch für Reste der Formel (VI-a) oder (VI-b).

Vorzugsweise stehen die Reste R^{10a} und/oder R^{10b} unabhängig voneinander für Wasserstoff, Methyl oder Phenyl, besonders bevorzugt jeweils einer der Reste R^{10a} und/oder R^{10b} für Methyl oder Phenyl und der zweite Rest R^{10a} und/oder R^{10b} sowie gegebenenfalls die beiden anderen Reste R^{10a} oder R^{10b} für Wasserstoff, oder ein Rest R^{10a} für Wasserstoff und der zweite Rest R^{10a} zusammen mit R¹¹ für Indandiyl. In bevorzugten Ausführungsformender vorliegenden Erfindung stehen R^{10a} in der Formel (VI-a) oder R^{10a} und R^{10b} in der Formel (VI-b) für Wasserstoff.

Vorzugsweise steht der Rest R¹¹ für Methyl, Isopropyl, tert.-Butyl, 2-Naphthyl, Phenyl, 2-Chlorphenyl, Benzyl, Hydroxymethyl, Tributylsiloxymethyl, 2-Tributylsiloxyethyl oder Methoxycarbonyl.

In Verbindungen der Formel (VII) steht R¹² bevorzugt für Wasserstoff, Hydroxy, C₁-C₄-Alkoxy und Phenyl, besonders bevorzugt für Wasserstoff.

Von den Resten X¹, X², X³ stehen bevorzugt mindestens zwei, bevorzugt drei für CH bzw CR¹². n steht bevorzugt für 0 oder 1 und besonders bevorzugt für einen Substituenten in 4-Stellung.

Bevorzugte Rutheniumkomplexe sind solche der Formel (VIII)

[Ru(V)(VII)] (VIII)

in der (V) für eine Verbindung der Formel (V) und (VII) für eine Verbindung der Formel (VII) steht. Solche Komplexe können in an sich bekannter Weise analog zur eingangs zitierten Literatur hergestellt werden.

Verbindungen der Formel (V) sind mit Ausnahme von Verbindungen in denen L¹ und L² jeweils für einen Rest der Formel (VI-a) stehen, worin R^{10a} jeweils für Wasserstoff und R¹¹ für Isopropyl oder Phenyl steht, bislang unbekannt und daher von der Erfindung mitumfasst. Das gleiche gilt für die in das erfindungsgemäße Verfahren eingesetzten und von solchen Verbindungen der Formel (V) abgeleiteten Rutheniumkomplexe und insbesondere Rutheniumkomplexe der Formel (VIII).

Beispiele für solche Verbindungen der Formel (V) sind
4-Chlor-2,6-bis[4'-(*S*)-phenyloxazolin-2'-yl]pyridin, 4-Phenyl-2,6-bis[4'-(*S*)-phenyloxazolin-2'-yl]pyridin, 2,6-bis[(*R*)-4'-(1''-naphthyl)-5',6'-dihydro-4'*H*-[1',3']oxazin-2'-yl]pyridin und 2,6-bis[(*R*)-4'-(2"-naphthyl)-5',6'-dihydro-4'*H*-[1',3']oxazin-2'-yl]pyridin.

Verbindungen der Formel (V) können beispielsweise gemäß Nishiyama et al., *Organometallics* **1991,** 10, 500 - 508 hergestellt werden.

Das erfindungsgemäße Verfahren wird in einer bevorzugten Ausführungsform in Gegenwart von organischem Lösungsmittel wie insbesondere sekundären oder tertiären Alkoholen, aprotisch polaren Lösungsmitteln, Ketonen, chlorierten Kohlenwasserstoffen und aromatischen Kohlenwasserstoffen durchgeführt. Unter aprotischen polaren Lösungsmittel sind solche zu verstehen, die bei 25°C eine Dielektrizitätskonstante von 5 oder mehr und einen pKs-Wert bezogen auf eine wässrige Bezugsskala bei 25°C von 20 oder mehr aufweisen. Für das erfindungsgemäße Verfahren besonders bevorzugt sind sekundäre und tertiäre Alkohole wie insbesondere *t*-Amylalkohol und *t*-Butylalkohol.

Die Reaktion wird beispielsweise so durchgeführt, dass die Verbindungen der Formel (III) und der Rutheniumkomplex sowie gegebenenfalls ein Additiv in einem organischen Lösungsmittel vorgelegt und mit dem Oxidationsmittel, welches gegebenenfalls in einem geeigneten organischen Lösungsmittel gelöst ist, versetzt werden. In einer bevorzugten Ausführungsform wird eine Lösung des Oxidationsmittels über einen Zeitraum von 10 Minuten bis 24 Stunden zum Reaktionsgemisch zudosiert.

Die Reaktionsdauer (Nachrührzeit) kann beispielsweise bis zu 24 Stunden, bevorzugt bis zu 5 Stunden und bevorzugt bis zu 2 Stunden betragen.

Die Reaktion kann bei Temperaturen von -20°C bis 150°C durchgeführt werden, bevorzugt bei 0 bis 80°C, besonders bevorzugt bei 0°C bis 40°C und ganz besonders bevorzugt bei 15°C bis 30°C.

Der Druck bei der Reaktion ist unkritisch und kann beispielsweise 0,5 bis 100 bar, bevorzugt 0,8 bis 10 bar betragen. Besonders bevorzugt ist Umgebungsdruck.

Als Verbindungen der Formel (IV) werden im Rahmen des erfindungsgemäßen Verfahrens besonders bevorzugt tert.-Butylhydroperoxid oder Wasserstoffperoxid eingesetzt. Das Oxidationsmittel wird bevorzugt in einer Menge von 1 bis 10 Moläquivalenten bezogen auf Verbindungen der Formel (III), besonders bevorzugt von 1 bis 5 Moläquivalenten und ganz besonders bevorzugt von 1 bis 3 Moläquivalenten eingesetzt. Das Oxidationsmittel wird gegebenenfalls vorteilhaft als Lösung in einem Lösungsmittel eingesetzt, besonders bevorzugt als Lösung in Wasser.

In einer weiteren Ausführungsform des erfindungsgemäßen Verfahrens können Additive zur Reaktionsmischung zugesetzt werden. Hierfür sind beispielsweise geeignet: Amine, Phosphite, Phosphanoxide, *N*-Methylmorpholin-*N*-oxid, 2,2,6,6-Tetramethylpiperidin-1-yloxyd, Pyridine, Pyridin-*N*-oxid, Imidazole, Chinolin, Chinolin-*N*-oxid, 2,2'-Bipyridyl, 2,2'-Bipyridyl-*N*,*N*'-dioxid, Ammoniumsalze, aromatische und aliphatische Carbonsäuren, Carbonsäureanhydride, (R)- oder (*S*)-Alkansulfinsäureamide oder aromatische Alkohole. Diese Additive werden bevorzugt in einer Menge von 5 bis 100 mol% bezogen auf Verbindungen der Formel (III), besonders bevorzugt von 10 bis 50 mol% und ganz besonders bevorzugt von 20 mol% eingesetzt.

Im Rahmen der Erfindung kann der Rutheniumkomplex entweder als isolierter Komplex eingesetzt werden oder in situ in der Reaktionsmischung erzeugt werden. In letzterem Falle werden eine geeigneter Rutheniumprecursorkomplex, wie z.B. [Ru(*p*-cymol)Cl₂]₂, und die beiden Liganden der Formeln (V) und (VII) in der Reaktionsmischung zusammengegeben.

Isolierte Komplexe sind beispielsweise
[Ru(4-Chlor-2,6-bis[4'-(*S*)-phenyloxazolin-2'-yl]pyridin)(Pyridin-2,6-dicarboxylat)]
[Ru(4-Phenyl-2,6-bis[4'-(*S*)-phenyloxazolin-2'-yl]pyridin) (Pyridin-2,6-dicarboxylat)]
[Ru(2,6-bis[4'-(*S*)-*iso*-propyl-oxazolin-2'-yl]pyridin)(Pyridin-2,6-dicarboxylat)]
[Ru(2,6-bis[(*R*)-4'-phenyl-5',6'-dihydro-4'*H*-[1',3']oxazin-2'-yl]pyridin)(Pyridin-2,6-dicarboxylat)]
[Ru(2,6-bis[(*R*)-4'-(1''-naphthyl)-5',6'-dihydro-4'*H*-[1',3']oxazin-2'-yl]pyridin)(Pyridin-2,6-dicarboxylat)]
[Ru(2,6-bis[(*R*)-4'-(2"-naphthyl)-5',6'-dihydro-4'*H*-[1',3']oxazin-2'-yl]pyridin)(Pyridin-2,6-dicarboxylat)]

Die Herstellung der isolierten Komplexe erfolgt vorzugsweise ebenfalls durch Zusammengeben eines geeigneten Rutheniumprecursorkomplexes, wie z.B. [Ru(*p*-cymol)Cl₂]₂, und der beiden Liganden der Formeln (V) und (VII) in der Weise, wobei beispielsweise der Rutheniumprecursorkomplex mit dem Liganden der Formel (V) in einem geeigneten Lösungsmittel in Inertgasatmosphäre vorgelegt und eine Lösung des Liganden der Formel (VII) zugegeben wird, die Reaktionsmischung anschließend erhitzt und der Rutheniumkomplex nach Aufarbeiten mittels Extraktion, Trocknen und gegebenenfalls anschließender chromatographischer Reinigung und/oder Umkristallisation isoliert wird.

Die Menge des eingesetzten Rutheniumkomplexes oder des eingesetzten Rutheniumprecursorkomplexes liegt im Rahmen der Erfindung beispielsweise zwischen 0.01 und 20 mol%, bevorzugt zwischen 1 und 10 mol% und besonders bevorzugt zwischen 2 und 5 mol%.

Auf erfindungsgemäße Weise können Verbindungen der Formel (I) in guten Ausbeuten mit hohen Stereoisomeren- bzw. Enantiomerenüberschüssen erhalten werden. Die Aufarbeitung kann in an sich bekannter Weise z.B. durch Quenchen mit Wasser, Extraktion mit einem geeigneten organischen Lösungsmittel und Destillation oder Umkristallisation des Epoxids erfolgen.

Die erfindungsgemäß herstellbaren Verbindungen der Formel (I) eignen sich insbesondere zur Herstellung von Arzneimitteln, Agrochemikalien, Polymeren oder Zwischenprodukten davon.

Das erfindungsgemäße Verfahren zeichnet sich dadurch aus, dass die asymmetrische Epoxidierung von Olefinen unter hoher Chemo- und Enantioselektivität verläuft und sehr gute Produktausbeuten liefert. Gleichzeitig ist die Möglichkeit der Verwendung des preisgünstigen Oxidationsmittels Wasserstoffperoxid ein besonderer Vorteil.

### Beispiele

### Allgemeine Arbeitsvorschrift:

In einem typischen Experiment werden ein Olefin (0.5 mmol) und der Rutheniumkomplex [Ru(*S*,*S*-2,6-Bis[4'-(*S*)-phenyloxazolin-2'-yl]pyridin)(Pyridin-2,6-dicarboxylat)] (0.025 mmol) mit *t*-Amylalkohol (9 ml) versetzt und gegebenenfalls ein Additiv zugesetzt. Zu dieser Mischung wird eine Lösung des Oxidationsmittels (0.75 mmol) in *t*-Amylalkohol (1 ml) innerhalb von 12 h dosiert. Nach Beendigung der Reaktion (Verfolgung des Umsatzes durch GC-FID) wird die Reaktionsmischung mit gesättigter Na₂SO₃-Lösung (ca. 10 ml) gequencht und zweimal mit Dichlormethan (je 10 ml) extrahiert. Nach destillativer Entfernung des Solvens wird das Produkt durch Säulenchromatographie gereinigt. Alle unten aufgeführten Produkte wurden durch GC-MS und NMR-Daten sowie Vergleiche mit authentischen Proben (GC-FID) bestätigt.

### Beispiel 1

Aus (*E*)-Stilben wurde mit H₂O₂ als Oxidationsmittel gemäß der allgemeinen Arbeitsvorschrift (*E*)-Stilbenoxid in 99 % Ausbeute und mit 67 % *ee* erhalten.

### Beispiel 2

Aus (*E*)-Stilben wurde mit H₂O₂ als Oxidationsmittel gemäß der allgemeinen Arbeitsvorschrift, jedoch in Gegenwart von 0.025 mmol 2,2'-Bipyridyl-*N*,*N*'-dioxid als Additiv Stilbenoxid in 96 % Ausbeute und mit 71 % *ee* erhalten.

### Beispiel 3

Aus (*E*)-Stilben wurde mit H₂O₂ als Oxidationsmittel gemäß der allgemeinen Arbeitsvorschrift, jedoch mit dem Katalysator [Ru(4-Chlor-2,6-bis[4'-(*S*)-phenyloxazolin-2'-yl]pyridin)(Pyridin-2,6-dicarboxylat)] (*E*)-Stilbenoxid in 93 % Ausbeute und mit 71 % *ee* erhalten.

### Beispiel 4

Aus (*E*)-Stilben wurde mit H₂O₂ als Oxidationsmittel gemäß der allgemeinen Arbeitsvorschrift, jedoch mit dem Katalysator [Ru(4-Phenyl-2,6-bis[4'-(*S*)-phenyloxazolin-2'-yl]pyridin) (Pyridin-2,6-dicarboxylat)] (0.025 mmol) (*E*)-Stilbenoxid in 97 % Ausbeute und mit 69 % *ee* erhalten.

### Beispiel 5

Aus (*E*)-Stilben wurde mit H₂O₂ als Oxidationsmittel gemäß der allgemeinen Arbeitsvorschrift, jedoch mit dem Katalysator [Ru(2,6-bis[4'-(*S*)-*iso*-propyl-oxazolin-2'-yl]pyridin)(Pyridin-2,6-dicarboxylat)] (0.025 mmol) (*E*)-Stilbenoxid in 100 % Ausbeute und mit 54 % *ee* erhalten.

### Beispiel 6

Aus Styrol wurde mit H₂O₂ als Oxidationsmittel analog zu Beispiel 1 Styroloxid in 71 % Ausbeute und mit 31 % *ee* erhalten.

### Beispiel 7

Aus Styrol wurde mit H₂O₂ als Oxidationsmittel und Essigsäure als analog zu Beispiel 1 Styroloxid in 75 % Ausbeute und mit 42 % *ee* erhalten.

### Beispiel 8

Aus *p*-Chlorstyrol wurde mit *t*-BuOOH als Oxidationsmittel analog zu Beispiel 1 *p*-Chlorstyroloxid in 48 % Ausbeute und mit 34 % *ee* erhalten.

### Beispiel 9

Aus *p*-Fluorstyrol wurde mit *t*-BuOOH als Oxidationsmittel analog zu Beispiel 1 *p*-Fluorstyroloxid in 54 % Ausbeute und mit 34 % *ee* erhalten.

### Beispiel 10

Aus α-Methylstyrol wurde mit H₂O₂ als Oxidationsmittel analog zu Beispiel 1 α-Methylstyroloxid in 52 % Ausbeute und mit 13 % *ee* erhalten.

### Beispiel 11

Aus β-Methylstyrol wurde mit H₂O₂ als Oxidationsmittel analog zu Beispiel 1 β-Methylstyroloxid in 82 % Ausbeute und mit 58 % *ee* erhalten.

### Beispiel 12

Aus 2-Methyl-1-phenyl-1-propen wurde mit *t*-BuOOH als Oxidationsmittel analog zu Beispiel 1 2-Methyl-1-phenyl-1-propenoxid in 95 % Ausbeute und mit 65 % *ee* erhalten.

### Beispiel 13

Aus 1,2-Diphenyl-1-propen wurde mit *t*-BuOOH als Oxidationsmittel gemäß der allgemeinen Arbeitsvorschrift 1,2-Diphenyl-1-propenoxid in 93 % Ausbeute und mit 22 % *ee* erhalten.

### Beispiel 14

Aus Triphenylethylen wurde mit *t*-BuOOH als Oxidationsmittel analog zu Beispiel 1 Triphenylethylenoxid in 90 % Ausbeute und mit 8 % *ee* erhalten.

### Beispiel 15

Aus 1-Phenylcyclohexan wurde mit H₂O₂ als Oxidationsmittel analog zu Beispiel 1 1-Phenylcyclohexanoxid in 87 % Ausbeute und mit 12 % *ee* erhalten.

### Beispiel 16

Aus (*E*)-Stilben wurde mit H₂O₂ als Oxidationsmittel analog zu Beispiel 1, jedoch durch Erzeugung des Katalysators *in situ* aus 0.0125 mol [Ru(*p*-cymol)Cl₂]₂, 0.025 mol 2,6-Bis[4'-(*S*)-phenyl=oxazolin-2'-yl]pyridin und 0.025 mol Pyridin-2,6-dicarbonsäure-dinatriumsalz, (*E*)-Stilbenoxid in 96 % Ausbeute und mit 61 % *ee* erhalten.

Tabellarische Zusammenfassung der Beispiele 1 bis 16 zur asymmetrischen Epoxidierung von Olefinen in Gegenwart von Rutheniumkomplexen

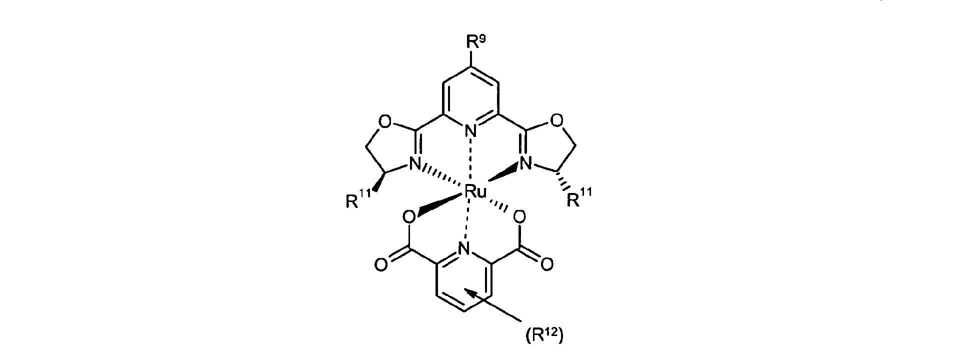

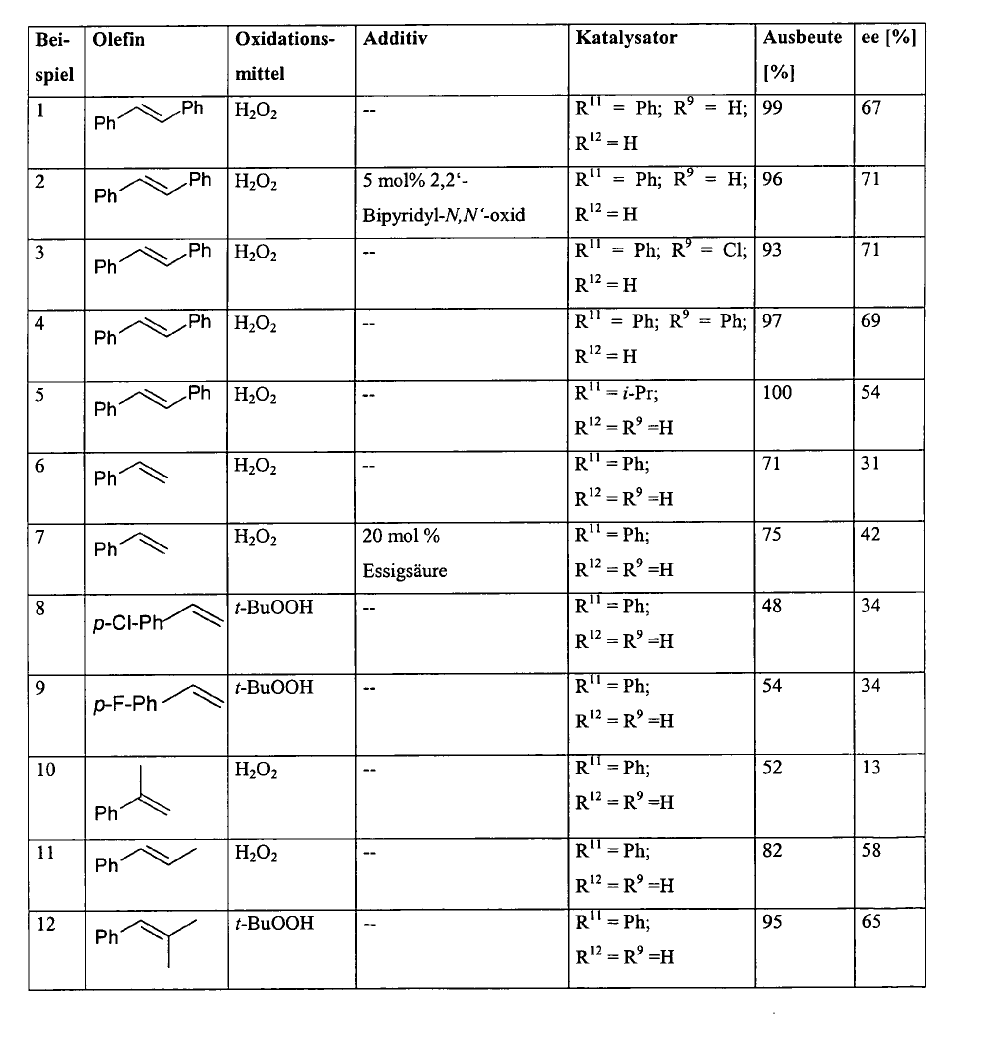

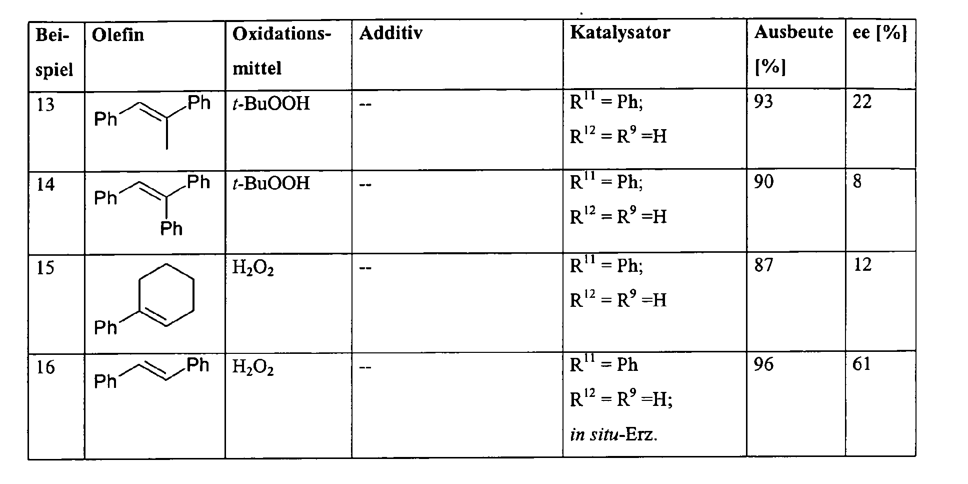

### Beispiel 17

Aus Styrol wurde mit H₂O₂ als Oxidationsmittel gemäß der allgemeinen Arbeitsvorschrift, jedoch mit dem Katalysator [Ru(2,6-bis[(*R*)-4'-phenyl-5',6'-dihydro-4'*H*-[1',3']oxazin-2'-yl]pyridin)(Pyridin-2,6-dicarboxylat)] (0.025 mmol) Styroloxid in 56 % Ausbeute und mit 48 % *ee* erhalten.

### Beispiel 18

Aus Styrol wurde mit H₂O₂ als Oxidationsmittel gemäß der allgemeinen Arbeitsvorschrift, jedoch mit dem Katalysator [Ru(2,6-bis[(*R*)-4'-(1"-naphthyl)-5',6'-dihydro-4'*H*-[1',3']oxazin-2'-yl]pyridin)(Pyridin-2,6-dicarboxylat)] (0.025 mmol) Styroloxid in 65 % Ausbeute und mit 38 % *ee* erhalten.

### Beispiel 19

### Herstellung von [Ru(2,6-bis[(R)-4'-(2"-naphthyl)-5',6'-dihydro-4'H-[1',3']oxazin-2'-yl]pyridin) (Pyridin-2,6-dicarboxylat)]

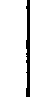

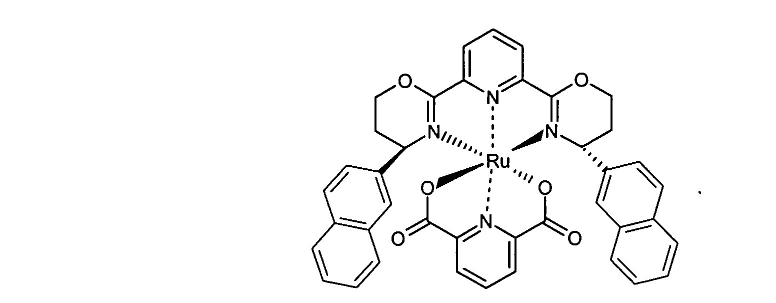

Zu einer Lösung von 150 mg 2,6-Bis[(*R*)-4'-(2"-naphthyl)-5',6'-dihydro-4'*H*-[1',3']oxazin-2'-yl]pyridin) (0,30 mmol) und 92 mg [Ru(*p*-cymol)Cl₂]₂ (0,15 mmol) in 2 ml Methanol wurde unter Ar-Atmosphäre eine Lösung von 64 mg Pyridin-2,6-dicarbonsäure-dinatriumsalz (0,30 mmol) in 2 ml einer Methanol/Wasser-(1:1)- Mischung getropft und die Reaktionsmischung für 1 h bei 65°C erhitzt. Anschließend wurde die Reaktionsmischung mit 30 ml CH₂Cl₂ extrahiert, die organische Phase mit 30 ml Wasser gewaschen, über MgSO₄ getrocknet, filtriert und unter reduziertem Druck das Lösungsmittel entfernt. Nach anschließender Chromatographie über Silicagel (70-230 mesh) mit CH₂Cl₂/Methanol (100:2 bis 100:5) als Gradienten-Elutionsmittel und erneutem Entfernen des Lösungsmittel unter reduziertem Druck wurde das Produkt in CH₂Cl₂/n-Hexan umkristallisiert. Es wurden 138 mg [Ru(2,6-bis[(*R*)-4'-(2"-naphthyl)-5',6'-dihydro-4'*H*-[1',3']oxazin-2'-yl]pyridin) (Pyridin-2,6-dicarboxylat)] (60% d.Th.) erhalten.

### Beispiel 20

Aus Styrol wurde mit H₂O₂ als Oxidationsmittel gemäß der allgemeinen Arbeitsvorschrift, jedoch mit dem Katalysator [Ru(2,6-bis[(*R*)-4'-(2"-naphthyl)-5',6'-dihydro-4'*H*-[1',3']oxazin-2'-yl]pyridin)(Pyridin-2,6-dicarboxylat)] (0.025 mmol) Styroloxid in 59 % Ausbeute und mit 48 % *ee* erhalten.

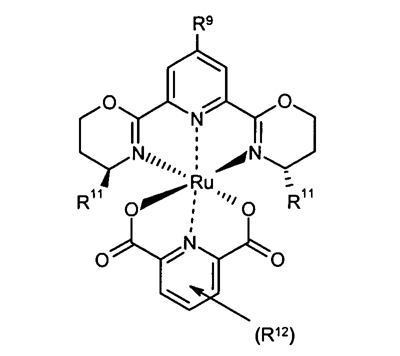

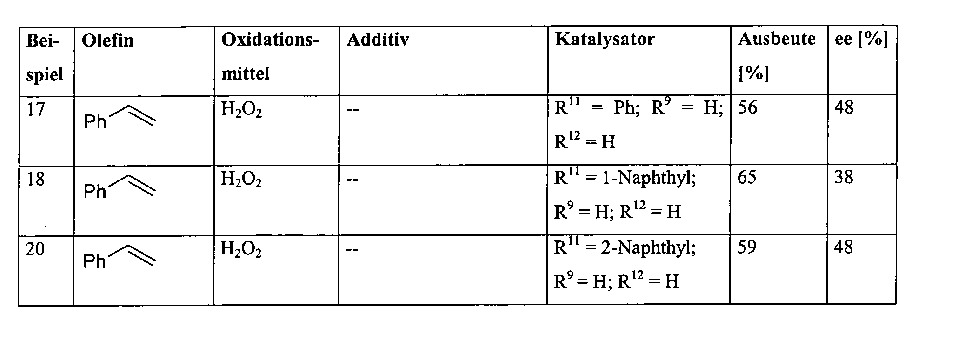

## Patentansprüche

1. Verfahren zur Herstellung von stereoisomerenangereicherten Verbindungen der Formel (I), 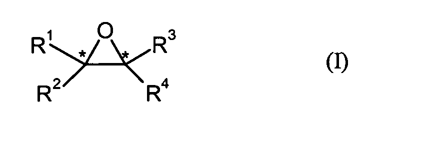 in der
"*" für ein (R)- oder (S)- konfiguriertes Kohlenstoffatom steht und R¹, R², R³ und R⁴ jeweils unabhängig voneinander für Wasserstoff, Alkyl, Aryl, Arylalkyl, Halogenalkyl oder Reste der Formel (IIa) bis (IIf) stehen
A-B-D-E (IIa) A-E (IIb)
A-SO₂-E (IIc) A-B-SO₂R⁶ (IId)
A-SO₃W (IIe) A-COW (IIf)
wobei in den Formeln (IIa) bis (IIf)
A fehlt oder für einen Alkylen- oder Halogenalkylenrest steht und
B fehlt oder für Sauerstoff oder NR⁵ steht, wobei
R⁵ für Wasserstoff, Arylalkyl oder Aryl steht und
D für eine Carbonyl-Gruppe steht und
E für R⁶, OR⁶, NHR⁷ oder N(R⁷)₂ steht,
wobei
R⁶ für Alkyl, Arylalkyl oder Aryl und
R⁷ jeweils unabhängig für Alkyl, Arylalkyl oder Aryl steht oder N(R⁷)₂ zusammen für einen cyclischen Aminorest mit 4 bis 12 Kohlenstoffatomen steht und
W für OH, NH₂, oder OM steht, wobei M ein Alkalimetallion, ein halbes Äquivalent eines Erdalkalimetallions, ein Ammoniumion oder ein organisches Ammoniumion steht
Oder jeweils zwei der Reste R¹, R², R³ und R⁴ zusammen Teil eines 3 bis 7-gliedrigen Cyclus sind, der insgesamt 3 bis 16 Kohlenstoffatome umfasst
**dadurch gekennzeichnet, dass** Verbindungen der Formel (III), 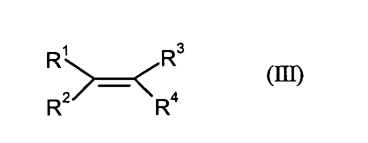 in der R¹, R², R³ und R⁴ jeweils unabhängig voneinander die vorstehend genannte Bedeutung besitzen,
mit Verbindungen der Formel (IV) umgesetzt werden,
R⁸-OOH (IV)
in der R⁸ für Wasserstoff, Alkyl oder Arylalkyl steht
wobei die Umsetzung in Gegenwart eines Rutheniumkomplexes erfolgt, der als Liganden sowohl Verbindungen der Formel (V) trägt 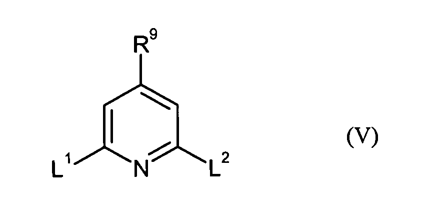 in der R⁹ für Wasserstoff, Halogen, Hydroxy, Alkoxy, Hydroxycarbonyl, Alkoxycarbonyl, Alkyl, Arylalkyl oder Aryl steht und
L¹ und L² jeweils unabhängig für Reste der Formel (VI-a) und/oder für Reste der Formel (VI-b) stehen 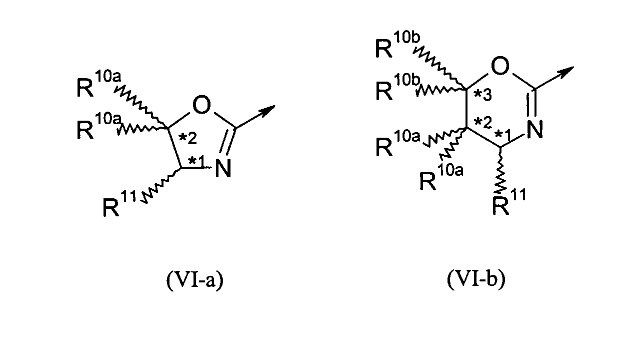 in der "*1" und/oder "*2" und/oder "*3" ein asymmetrisches Kohlenstoffatom in (R) oder (S)-Konfiguration ist, der Pfeil die Bindungsstelle zum zentralen Pyridincyclus zeigt und R^{10a}, R^{10b} und R¹¹ unabhängig voneinander für Alkyl, Alkoxyalkyl, Trialkylsiloxyalkyl, Alkoxycarbonyl, Arylalkyl oder Aryl steht oder R^{10a} und R¹¹ oder R^{10a} und R^{10b} Teil eines cyclischen Restes mit insgesamt 5 bis 16 Kohlenstoffatomen sind und R¹¹ und/oder R^{10a} und/oder R^{10b} darüber hinaus auch für Wasserstoff stehen kann
als auch Verbindungen der Formel (VII) trägt 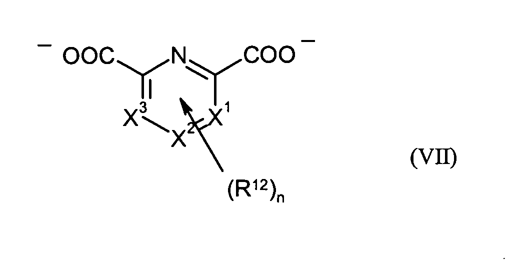 in der
X¹, X² und X³ jeweils unabhängig voneinander für N, CH bzw. CR¹² stehen und
R¹² für Wasserstoff, Halogen, Hydroxy, Hydroxycarbonyl, Alkoxycarbonyl, Alkoxy, Alkoxyalkyl, Arylalkyl oder Aryl steht und
n für =1, 2 oder 3, bevorzugt für 0, oder 1 und besonders bevorzugt für 0 steht.

2. Verfahren nach Anspruch 1, **dadurch gekennzeichnet, dass** in Formel (I) R¹, R², R³ und R⁴ bevorzugt jeweils unabhängig voneinander für Wasserstoff, C₁-C₈-Alkyl, C₅-C₁₄-Aryl, C₆-C₁₅-Arylalkyl, C₁-C₈-Halogenalkyl stehen oder jeweils zwei der Reste R¹, R², R³ und R⁴ zusammen Teil eines 3 bis 7-gliedrigen Cyclus sind, der insgesamt 3 bis 16 Kohlenstoffatome umfasst.

3. Verfahren nach Anspruch 1 oder 2, **dadurch gekennzeichnet, dass** in Formel (IV) R⁸ für Wasserstoff oder C₃-C₆-Alkyl steht.

4. Verfahren nach einem der Ansprüche 1 bis 3, **dadurch gekennzeichnet, dass** in Formel (IV) R⁸ für Wasserstoff steht.

5. Verfahren nach einem der Ansprüche 1 bis 4, **dadurch gekennzeichnet, dass** als Rutheniumkomplexe solche der Formel (VIII) eingesetzt werden
[Ru(V)(VII)] (VIII)
in der (V) für eine Verbindung der Formel (V) und (VII) für eine Verbindung der Formel (VII) steht oder solche die in situ in der Reaktionsmischung aus einem geeigneten Rutheniumprecursorkomplex und den beiden Liganden der Formeln (V) und (VII) in der Reaktionsmischung erzeugt werden.

6. Verfahren nach einem der Ansprüche 1 bis 5, **dadurch gekennzeichnet, dass** es in Gegenwart von sekundären oder tertiären Alkoholen als Lösungsmittel durchgeführt wird.

7. Verfahren nach einem der Ansprüche 1 bis 6, **dadurch gekennzeichnet, dass** Additive ausgewählt aus der Gruppe Amine, Phosphite, Phosphanoxide, *N*-Methylmorpholin-*N*-oxid, 2,2,6,6-Tetramethylpiperidin-1-yloxyd, Pyridine, Pyridin-*N*-oxid, Imidazole, Chinolin, Chinolin-*N*-oxid, 2,2'-Bipyridyl, 2,2'-Bipyridyl-*N*,*N*'-dioxid, Ammoniumsalze, aromatische und aliphatische Carbonsäuren, Carbonsäureanhydride, (*R*)- oder (*S*)-Alkansulfinsäureamide oder aromatische Alkohole zugesetzt werden.

8. Verbindungen der Formel (V) mit der Definition gemäß Anspruch 1 mit Ausnahme solcher in denen L¹ und L² jeweils für einen Rest der Formel (VI-a) stehen, worin R^{10a} jeweils für Wasserstoff und R¹¹ für Isopropyl oder Phenyl steht.

9. 4-Chlor-2,6-bis[4'-(*S*)-phenyloxazolin-2'-yl]pyridin, 4-Phenyl-2,6-bis[4'-(*S*)-phenyloxazolin-2'-yl]pyridin, 2,6-bis[(*R*)-4'-(1"-naphthyl)-5',6'-dihydro-4'*H*-[1',3']oxazin-2'-yl]pyridin und 2,6-bis[(*R*)-4'-(2"-naphthyl)-5',6'-dihydro-4'*H*-[1',3']oxazin-2'-yl]pyridin.

10. Rutheniumkomplexe enthaltend Verbindungen nach einem der Ansprüche 8 oder 9 und Verbindungen der Formel (VII) gemäß Definition in Anspruch 1.

11. Verwendung von Verbindungen der Formel (I), die nach einem oder mehreren der Ansprüche 1 bis 8 hergestellt werden zur Herstellung von Arzneimitteln, Agrochemikalien, Polymeren oder Zwischenprodukten davon.
